# EUROPÄISCHE PATENTSCHRIFT

(11) **EP 3 200 847 B1**
(45) Veröffentlichungstag und Bekanntmachung des Hinweises auf die Patenterteilung: **03.07.2019**
(21) Anmeldenummer: 15766755.1
(22) Anmeldetag: 18.09.2015
(51) Int. Cl.: A61M 1/16, A61M 1/34, A61M 1/36

(54) **VERFAHREN ZUR IDENTIFIZIERUNG EINES FILTERS**
METHOD FOR IDENTIFYING A FILTER
PROCEDE D'IDENTIFICATION D'UN FILTRE

(30) Priorität: 29.09.2014 DE 102014014418
(43) Veröffentlichungstag der Anmeldung: 09.08.2017
(73) Patentinhaber: Fresenius Medical Care Deutschland GmbH, 61352 Bad Homburg (DE)
(72) Erfinder: THYS, Martin, 97508 Grettstadt (DE); NOACK, Joachim, 97616 Bad Neustadt (DE)
(74) Vertreter: Herrmann, Uwe
(86) Internationale Anmeldenummer: PCT/EP2015/001867
(87) Internationale Veröffentlichungsnummer: WO 2016/050339

(56) Entgegenhaltungen:
- EP-A1- 1 892 000
- WO-A2-03/092871
- DE-A1- 19 534 417
- FR-A1- 2 936 713

## Beschreibung

Die vorliegende Erfindung betrifft ein Verfahren zur Identifizierung eines Filters, wobei der Filter wenigstens eine Retentatseite und wenigstens eine Permeatseite aufweist, die durch wenigstens ein Filtermedium voneinander getrennt sind.

Bei vielen Vorrichtungen zur Blutbehandlung kommen Filter mit semipermeablen Membranen zum Einsatz. Bei solchen Verfahren kann es sich beispielsweise um die Hämofiltration, Hämodialyse, Hämodiafiltration, Apherese, eine Medikation etc. handeln.

Häufig kommen bei den Blutbehandlungen derselben Art unterschiedliche Filtertypen zur Anwendung.

Da der Erfolg der Behandlung wesentlich von dem verwendeten Filtertyp abhängt, ist unbedingt sicherzustellen, dass für die jeweilige Behandlung der richtige Filter zur Anwendung kommt.

Üblich ist es, dass der Bediener eines Blutbehandlungsgerätes einen Filter auswählt und diesen zur Behandlung einsetzt. Dabei kann jedoch der Fall eintreten, dass aufgrund eines Irrtums ein falscher bzw. ein ungeeigneter Filter eingesetzt wird.

Bei aus dem Stand der Technik bekannten Verfahren wird ein Fehler bei der Auswahl des Filters dadurch ausgeschlossen, dass die Behandlungsvorrichtung, in die der Filter eingesetzt wird, den Typ des Filters anhand von äußerlich am Filter vorhandenen Merkmalen, wie beispielsweise mechanischen oder optischen Merkmalen bestimmt, wodurch eine Behandlung mit einem unpassenden Filter verhindert werden kann.

Die Erfassung dieser äußeren Merkmale eines Filters bedarf des Einsatzes wenigstens eines Sensors oder dergleichen, der speziell für diese Filtererkennung eingesetzt wird. WO03/092871 A2 offenbart ein Verfahren zur Identifizierung eines Filters anhand eines Transponders.

Aus dem Stand der Technik ist es des Weiteren bekannt, die Ultrafiltrationskonstante eines Filters mithilfe von mindestens zwei Drucksensoren in den hydraulischen Fluidkreisläufen, mit denen der Filter verbunden ist, zu bestimmen. Aufgrund der Auswertung der Signale der zwei Drucksensoren kann auf den Filtertyp geschlossen werden bzw. die Ultrafiltrationskonstante gemessen werden. Diese Auswertung der Signale von zwei oder mehr als zwei Drucksensoren in aufeinanderfolgenden Zuständen des Verfahrens ist jedoch mit dem Nachteil eines vergleichsweise hohen technischen und zeitlichen Aufwandes verbunden.

Der vorliegenden Erfindung liegt daher die Aufgabe zugrunde, ein Verfahren der eingangsgenannten Art dahingehend weiterzubilden, dass dieses einfach durchgeführt werden kann und einen geringen technischen Aufwand mit sich bringt.

Diese Aufgabe wird durch ein Verfahren mit den Merkmalen des Anspruchs 1 gelöst. Danach ist vorgesehen, dass das Verfahren das Erzeugen eines Druckes in einem Fluid, insbesondere in einer Flüssigkeit, wie z. B. in einer Dialyselösung auf der Retentatseite oder auf der Permeatseite des Filters mittels einer Druckquelle, wie beispielsweise einer Pumpe umfasst. Das Verfahren umfasst des Weiteren das Abschalten der Druckquelle sowie die sich daran anschließende Messung des zeitlichen Druckverlaufes in dem Fluid. Die Druckmessung findet ausschließlich nach dem Abschalten der Druckquelle oder vor und nach dem Abschalten der Druckquelle statt.

Durch die Messung des Druckverlaufes über die Zeit lässt sich der Ultrafiltrationskoeffizient und/oder der Flusswiderstand des jeweils verwendeten Filtertyps auf vergleichsweise einfache Art und Weise ermitteln. Wird ein Filter eingesetzt, der einen geringen Flusswiderstand bzw. einen hohen Ultrafiltrationskoeffizienten aufweist, sinkt der Druck nach Abschalten der Druckquelle innerhalb eines bestimmten Zeitraumes nur relativ wenig ab, da bereits während des Druckaufbaus ein vergleichsweise großes Fluidvolumen über das Filtermedium übergetreten ist und somit auf dieser Seite ebenfalls bereits ein Druckaufbau erfolgt ist.

Wird hingegen ein Filter mit einem hohen Flusswiderstand bzw. mit einem kleinen Ultrafiltrationskoeffizienten verwendet, stellt sich während des Druckaufbaus mittels der Druckquelle auf der anderen Seite des Filtermediums ein relativ geringer Überdruck ein, da das Übertreten von Fluid entsprechend behindert wird. Als Folge sinkt der Druck auf der Seite, auf der die Druckquelle angeschlossen ist nach dem Abschalten der Druckquelle relativ stark ab, da bis zum vollständigen Druckausgleich über das Filtermedium noch eine relativ große Fluidmenge über das Filtermedium verschoben wird.

Somit ist es möglich, durch die Messung des Druckverlaufes Rückschlüsse auf den Filtertypen zu ziehen bzw. auf den Ultrafiltrationskoeffizienten bzw. auf den Flusswiderstand des angeschlossenen Filtertyps.

Der Begriff der "Messung des Druckverlaufes" in dem Fluid über die Zeit umfasst sowohl die Messung von mehreren Druckwerten als auch die Messung nur eines einzigen Druckwertes nach dem Abschalten der Druckquelle. Eine besonders zuverlässige Aussage über den Filtertyp ist allerdings eher möglich, wenn mehrere Druckwerte nach dem Abschalten der Druckquelle in zeitlicher Abfolge genommen werden.

Das Verfahren lässt sich bei allen Geräten anwenden, bei deren Funktionsweise ein Filter mit einem Filtermedium, insbesondere mit einer semipermeablen Membran zum Einsatz kommt.

Als Filter kommt beispielsweise ein Dialysator in Betracht, wie er bei einem Dialysegerät eingesetzt wird. Allerdings ist der Begriff "Filter" nicht auf einen Dialysator beschränkt, sondern umfasst auch beliebige andere Filter, vorzugsweise solche, die zum Einsatz in einem Blutbehandlungsgerät geeignet und bestimmt sind.

In einer weiteren Ausgestaltung der Erfindung ist vorgesehen, dass der Druckverlauf nur mittels eines einzigen Drucksensors gemessen wird. Dabei ist vorzugsweise vorgesehen, dass der Druck auf der Seite des Filters gemessen wird, auf der auch der Druck durch die Druckquelle erzeugt wurde. Dies bedeutet, dass Druckquelle und Drucksensor in dieser Ausgestaltung der Erfindung auf derselben Seite (Permeatseite oder Retentatseite) des Filters angeordnet sind.

Weiterhin kann vorgesehen sein, dass die Druckquelle so lange den Druck erhöht, bis ein bestimmter Druck erreicht ist und die Druckquelle sodann abgeschaltet wird. Im Anschluss daran kann die Aufzeichnung des Druckes bzw. die Erfassung von Druckwerten über die Zeit erfolgen, auf deren Grundlage sodann auf den Filtertypen geschlossen werden kann.

In einer weiteren Ausgestaltung des Verfahrens ist vorgesehen, dass die Retentatseite und/oder die Permeatseite des Filters mit einer oder mehreren Fluidleitungen verbunden sind, wobei in diesen Fluidleitungen eine oder mehrere Absperrventile vorgesehen sind und wobei diese Absperrventile während der Messung des Druckverlaufes in dem Fluid über die Zeit geschlossen sind. Vorzugsweise erfolgt die Messung des Druckverlaufes über die Zeit somit in einem geschlossenen System, in dem gemessen wird, wie sich der Druck auf der Seite des Filters, die nicht unmittelbar mit der Druckquelle in Fluidverbindung steht, sondern von dieser mittels des Filtermediums getrennt ist, über die Zeit ändert.

Denkbar ist es, dass die Retentatseite und die Permeatseite jeweils Bestandteile eines Dialyse- bzw. Blutkreislaufes sind, das heißt von Kreisläufen, die im Betrieb der Blutbehandlungsvorrichtung einerseits von Dialyselösung und andererseits von Blut durchströmt werden. Die genannten Fluidleitungen stellen in diesem Falle somit Bestandteile eines extrakorporalen Kreislaufes bzw. Bestandteile eines Kreislaufes für die Dialyselösung dar.

Vorzugsweise sind somit insgesamt vier Leitungen vorgesehen, wobei jeweils eine Zuleitung und eine Ableitung für die Retentatseite und für die Permeatseite des Filters vorgesehen sind. Denkbar ist es, dass in einer dieser Leitungen die Pumpe bzw. Druckquelle angeordnet ist und alle anderen Leitungen mit wenigstens einem Absperrventil versehen sind, wobei diese während der Druckmessung und bei stehender Pumpe bzw. abgeschalteter Druckquelle geschlossen sind.

In einer weiteren bevorzugten Ausgestaltung der Erfindung ist vorgesehen, dass der Filter vor dem Aufbringen des Druckes vollständig mit Fluid gefüllt wird.

Die vorliegende Erfindung betrifft des Weiteren ein System zur Identifizierung eines Filters, wobei der Filter wenigstens eine Retentatseite und wenigstens eine Permeatseite aufweist, die durch wenigstens ein Filtermedium voneinander getrennt sind. Des Weiteren umfasst das System wenigstens eine Druckquelle, insbesondere wenigstens eine Pumpe, die derart ausgebildet ist, dass in einem Fluid, insbesondere in einer Flüssigkeit auf der Retentatseite oder auf der Permeatseite des Filters ein Druck erzeugbar ist. Des Weiteren ist wenigstens ein Drucksensor vorgesehen, der den Druckverlauf in dem Fluid über die Zeit erfasst, sowie wenigstens eine Steuereinheit, die sowohl mit der Druckquelle als auch mit dem Drucksensor so in Verbindung steht, dass die Messung des Druckverlaufes über die Zeit ausschließlich oder auch nach dem Abschalten der Druckquelle erfolgt.

Der Begriff "Abschalten der Druckquelle" umfasst beispielsweise das Abschalten einer Pumpe oder auch das Abkoppeln des Filters von einer sonstigen Druckquelle, beispielsweise durch Absperren eines Ventils.

In einer weiteren Ausgestaltung der Erfindung ist vorgesehen, dass es sich bei dem Filter um einen Dialysator handelt und/oder dass es sich bei dem Filtermedium um eine semipermeable Membran handelt.

Wie bereits oben ausgeführt besteht eine bevorzugte Ausgestaltung der Erfindung darin, dass nur ein einziger Drucksensor vorhanden ist, auf dessen Grundlage bzw. mit dessen Messwerten der Filtertyp bestimmbar ist. Vorzugsweise befindet sich dieser Drucksensor auf der Seite des Filters, auf der der Druck durch die Druckquelle erzeugt wurde.

Eine besonders einfache Ausgestaltung der Erfindung ergibt sich dann, wenn kein weiterer Sensor zur Bestimmung des Filtertyps vorgesehen ist und insbesondere wenn kein Sensor zur Erfassung von mechanisch oder optisch erfassbaren Filtermerkmalen vorhanden ist.

Die Steuereinheit ist so ausgebildet, dass diese die Druckquelle derart betreibt, dass der Druck in dem Fluid erhöht wird, bis ein bestimmter Druck erreicht ist und die Druckquelle anschließend abschaltet. Sodann erfolgt die Messung des Druckes über die Zeit. Auf der Grundlage dieser Messung wird dann auf den Filtertyp bzw. die Filtercharakteristik geschlossen. In einer weiteren Ausgestaltung der Erfindung ist vorgesehen, dass die Retentatseite und/oder die Permeatseite des Filters mit einer oder mehreren Fluidleitungen verbunden ist, wobei in diesen Fluidleitungen eine oder mehrere Absperrventile angeordnet sind. Dabei kann die Steuereinheit derart ausgebildet sein, dass sie diese Absperrventile während der Messung des Druckverlaufes in dem Fluid über die Zeit schließt. Wie bereits oben ausgeführt, ergibt sich dadurch ein insgesamt geschlossenes System, bei dem die Zufuhr und Abfuhrleitungen der Retentatseite und der Permeatseite durch Absperrventile bzw. die Pumpe oder eine sonstige Druckquelle abgesperrt sind. Eine Fluidbewegung in diesem abgeschlossenen System ist nur über das Filtermedium hindurch möglich. Diese Fluidbewegung führt zu der messbaren Druckänderung.

Die vorliegende Erfindung betrifft des Weiteren ein Blutbehandlungsgerät, insbesondere ein Dialysegerät, das wenigstens ein System gemäß der Erfindung umfasst.

Vorzugsweise ist die Druckquelle bzw. die Pumpe sowie der Drucksensor der Vorrichtung ein Bestandteil eines derartigen Blutbehandlungsgerätes, wobei diese Bestandteile, das heißt die Druckquelle und der Drucksensor in einer bevorzugten Ausgestaltung ohnehin an dem Blutbehandlungsgerät vorhanden sind, sodass kein gesondertes Equipment zur Durchführung des Verfahrens erforderlich ist.

Denkbar ist es beispielsweise, dass es sich bei der Druckquelle um die Pumpe handelt, die Blut oder die Dialyselösung im Betrieb des Blutbehandlungsgerätes fördert und dass es sich bei dem Drucksensor um einen ohnehin vorhandenen Drucksensor zur Messung des Druckes auf der Blutseite oder Dialysatseite des Blutbehandlungsgerätes handelt.

Weitere Einzelheiten und Vorteile der Erfindung werden anhand eines in der Zeichnung dargestellten Ausführungsbeispiels näher erläutert.

Es zeigen:
- Figur 1:: Eine schematische Ansicht eines Systems gemäß der vorliegenden Erfindung zur Identifizierung eines Filters und
- Figur 2:: zeitliche Verläufe des Druckes beim Druckaufbau sowie nach dem Abschalten der Druckquelle für unterschiedliche Filtertypen.

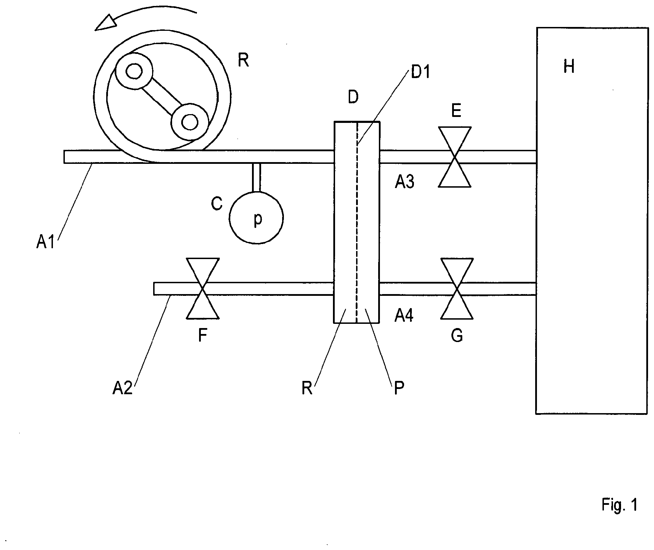

In Figur 1 ist mit dem Bezugszeichen A1 eine Leitung bzw. ein Patientenanschluss gekennzeichnet, die/der im Betrieb eines Blutbehandlungsgerätes mit dem Patienten in Verbindung steht und beispielsweise Blut von dem Patienten in einem extrakorporalen Kreislauf zu dem Dialysator D führt. In diesem extrakorporalen Kreislauf befindet sich die Pumpe B sowie der Dialysator D, der durch einen semipermeable Membran D1 in eine Retentatseite R und in eine Permeatseite P getrennt ist.

Das Bezugszeichen A2 kennzeichnet eine zweite Leitung des extrakorporalen Blutkreislaufes bzw. einen Patientenanschluss, durch den im Betrieb der Vorrichtung das Retentat zurück zum Patienten gefördert wird. In der Leitung A2 befindet sich wie aus Figur 1 ersichtlich eine Schlauchklemme F, mittels derer die Leitung A2 abgesperrt werden kann.

Wie dies weiter aus Figur 1 hervorgeht, ist die Permeatseite P des Filters D ebenfalls mit einer Zuleitung A4 sowie mit einer Ableitung A3 verbunden. Die Leitungen A4 und A3 weisen ebenfalls Schlauchklemmen G und E auf, mittels derer diese Leitungen abgesperrt werden können. Das Bezugszeichen H kennzeichnet die Hydraulik des Systems, bei der es sich beispielsweise um die Versorgung eines Blutbehandlungsgerätes mit Dialyselösung, um Pumpen zur Förderung der Dialyselösung etc. handeln kann. Im Behandlungsbetrieb wird durch die Leitung A4 die Dialyselösung zum Filter D hin transportiert und durch die Leitung A3 die Dialyselösung vom Filter D abgeleitet.

Die Systemschläuche A1 und A2 besitzen eine begrenzte Steifigkeit, stellen somit ein druckabhängiges Volumen dar.

Wie dies weiter aus Figur 1 hervorgeht, befindet sich zwischen der Pumpe B und dem Filter D ein Drucksensor C zur Messung des Druckes p.

Das Verfahren zur Identifizierung des Filters gestaltet sich wie folgt:
Die Pumpe B fördert, bis sich am Drucksensor C, der zwischen Pumpe P und Filter D angeordnet ist, ein bestimmter, zuvor gewählter Druck einstellt. Dabei kann es sich beispielsweise um einen Relativdruck von 1 bar handeln.

Wird dieser Druck erreicht, wird dies durch eine Steuereinheit erkannt und die Pumpe B wird abgeschaltet. Bis zum Zeitpunkt des Abschaltens der Pumpe B stellt sich durch einen Fluss über die Membran D1 im Filter D in den Systemschläuchen A3, A4 ein Druck ein. Dieser Druck ist abhängig vom Flusswiderstand der Membran D1. Je geringer der Flusswiderstand der Membran ist, desto mehr Flüssigkeit strömt während des Betriebs der Pumpe B über die Membran in die Leitungen A3 und A4.

Nach dem Abschalten der Pumpe B erfolgt ein Druckausgleich über die Membran D1, dessen Geschwindigkeit vom Filtertyp bzw. von der Eigenschaft des Filtermediums abhängt. Hierbei wird Flüssigkeit in die Systemschläuche A3 und A4 verschoben.

Bei einer Membran mit einem geringen Flusswiderstand bzw. einem hohen Ultrafiltrationskoeffizienten stellt sich bereits während des Betriebes der Pumpe D in den Schläuchen A3 und A4 ein relativ hoher Überdruck ein, da bereits eine vergleichsweise große Flüssigkeitsmenge über die Membran D1 übertritt. Dies hat zur Folge, dass der Druck p am Drucksensor C nach dem Abschalten der Pumpe innerhalb eines zuvor definierten Zeitraums, beispielsweise < 5 Sekunden nur relativ wenig absinkt.

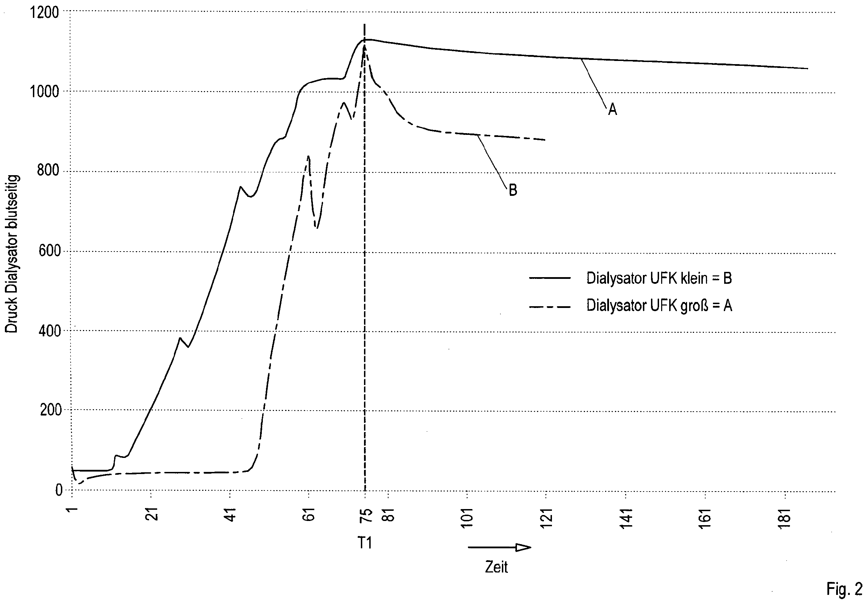

Dies ist anhand der Linie A in Figur 2 erkennbar. Bis zu dem Zeitpunkt T1 wird der Druck durch die Pumpe erhöht. Sodann wird die Pumpe abgeschaltet und die Klemmen E, F und G werden geschlossen. Wie diese aus dem Druckverlauf A hervorgeht, sinkt nach dem Abschalten der Pumpe, das heißt nach dem Zeitpunkt T1 der Druck nur um einen relativ geringen Betrag, typischerweise um weniger als 30 mm Hg, da bis zum vollständigen Druckausgleich über die Membran nur noch eine relativ geringe Flüssigkeitsmenge über die Membran verschoben wird.

Bei einer Membran mit einem hohen Flusswiderstand bzw. kleinem Ultrafiltrationskoeffizienten ergibt sich ein abweichender Druckverlauf, wie dies anhand der Linie B in Figur 2 erkennbar ist.

Bei einer Membran mit einem solchen hohen Flusswiderstand stellt sich während des Förderns der Pumpe B in den Systemschläuchen A3 und A4 ein relativ geringer Überdruck ein, da nur vergleichsweise wenig Flüssigkeit über die Membran D1 übertritt.

Nach dem Abschalten der Pumpe zum Zeitpunkt T1 sinkt in der Folge der Druck p am Drucksensor C in dem genannten Zeitraum typischerweise von < 5 Sekunden relativ stark ab. Typischerweise liegt der Druckabfall bei über 100 mm Hg, wie dies aus der Linie B erkennbar ist. Dies ist darauf zurückzuführen, dass bis zum vollständigen Druckausgleich über der Membran noch eine relativ große Flüssigkeitsmenge über die Membran verschoben wird, was zu einem entsprechenden Druckabfall in dem Kompartiment des Filters führt, aus dem die Flüssigkeit verschoben wird.

In dem Ausführungsbeispiel befindet sich die Pumpe B sowie der Drucksensor C auf der Blutseite. Grundsätzlich ist jedoch ebenfalls von der Erfindung umfasst, Pumpe und Sensor auf der Dialysatseite anzuordnen.

## Patentansprüche

1. Verfahren zur Identifizierung eines Filters, wobei der Filter wenigstens eine Retentatseite und wenigstens eine Permeatseite aufweist, die durch wenigstens ein Filtermedium voneinander getrennt sind, wobei das Verfahren das Erzeugen eines Druckes in einem Fluid, insbesondere in einer Flüssigkeit, auf Retentatseite oder auf der Permeatseite mittels wenigstens einer Druckquelle, insbesondere mittels wenigstens einer Pumpe, das Abschalten der Druckquelle sowie die sich an das Abschalten der Druckquelle anschließende Messung des Druckverlaufes in dem Fluid über die Zeit umfasst.

2. Verfahren nach Anspruch 1, **dadurch gekennzeichnet, dass** es sich bei dem Filter um einen Dialysator handelt und/oder dass es sich bei dem Filtermedium um eine semipermeable Membran handelt.

3. Verfahren nach einem der vorhergehenden Ansprüche, **dadurch gekennzeichnet, dass** der Druckverlauf nur mittels eines einzigen Drucksensors gemessen wird, wobei vorzugsweise vorgesehen ist dass der Druck auf der Seite (Permeatseite oder Retentatseite) des Filters gemessen wird, auf der der Druck durch die Druckquelle erzeugt wurde.

4. Verfahren nach einem der vorhergehenden Ansprüche, **dadurch gekennzeichnet, dass** die Druckquelle solange den Druck in dem Fluid erhöht, bis ein bestimmter Druck erreicht ist und dass die Druckquelle anschließend abgeschaltet wird.

5. Verfahren nach einem der vorhergehenden Ansprüche, **dadurch gekennzeichnet, dass** die Retentatseite und/oder die Permeatseite des Filters mit einer oder mehreren Fluidleitungen verbunden ist, wobei in diesen Fluidleitungen ein oder mehrere Absperrventile angeordnet sind und dass diese Absperrventile während der Messung des Druckverlaufes in dem Fluid über die Zeit geschlossen sind.

6. Verfahren nach einem der vorhergehenden Ansprüche, **dadurch gekennzeichnet, dass** der Filter vor dem Aufbringen des Druckes vollständig mit Fluid gefüllt wird.

7. System zur Identifizierung eines Filters, wobei der Filter wenigstens eine Retentatseite und wenigstens eine Permeatseite aufweist, die durch wenigstens ein Filtermedium voneinander getrennt sind, wobei wenigstens eine Druckquelle, insbesondere wenigstens eine Pumpe vorgesehen ist, die derart ausgebildet und angeordnet ist, dass in einem Fluid, insbesondere in einer Flüssigkeit, auf Retentatseite oder auf der Permeatseite des Filters ein Druck erzeugbar ist, sowie mit wenigstens einem Drucksensor, der derart angeordnet ist, dass mittels des Drucksensors der Druckverlauf in dem Fluid über die Zeit ermittelbar ist, wobei das System des Weiteren wenigstens eine Steuereinheit aufweist, die mit der Druckquelle sowie mit dem Drucksensor in Verbindung steht,
**dadurch gekennzeichnet, dass** die Steuereinheit dazu ausgelegt ist:
die Druckquelle so anzusteuern, dass der Druck in dem Fluid erhöht wird, bis ein bestimmter Druck erreicht ist und die Druckquelle anschließend abzuschalten,
eine Messung eines Druckverlaufes über die Zeit nach dem Abschalten der Druckquelle vorzunehmen, und
auf Grundlage dieser Messung auf einen Filtertyp bzw. eine Filtercharakteristik zu schließen.

8. System nach Anspruch 7, **dadurch gekennzeichnet, dass** es sich bei dem Filter um einen Dialysator handelt und/oder dass es sich bei dem Filtermedium um eine semipermeable Membran handelt.

9. System nach Anspruch 7 oder 8, **dadurch gekennzeichnet, dass** nur eine einziger Drucksensor vorhanden ist, wobei vorzugsweise vorgesehen ist, dass dieser Drucksensor auf der Seite (Permeatseite oder Retentatseite) des Filters angeordnet ist, auf der der Druck durch die Druckquelle erzeugbar ist.

10. System nach einem der Ansprüche 7 bis 9, **dadurch gekennzeichnet, dass** kein Sensor zur Erfassung von mechanisch oder optisch erfassbaren Filtermerkmalen vorhanden ist.

11. System nach einem der Ansprüche 7 bis 10, **dadurch gekennzeichnet, dass** die Retentatseite und/oder die Permeatseite des Filters mit einer oder mehreren Fluidleitungen verbunden ist, wobei in diesen Fluidleitungen ein oder mehrere Absperrventile angeordnet sind und wobei die Steuereinheit derart ausgebildet ist, dass sie diese Absperrventile während der Messung des Druckverlaufes in dem Fluid über die Zeit schließt.

12. System nach Anspruch 11, **dadurch gekennzeichnet, dass** die Retentatseite sowie die Permeatseite jeweils mit einer Zu- und mit einer Ablaufleitung verbunden sind, dass die Druckquelle in einer dieser Leitungen angeordnet ist und dass sich in den weiteren Leitungen die genannten Absperrventile befinden.

13. Blutbehandlungsgerät, insbesondere Dialysegerät mit wenigstens einem System gemäß einem der Ansprüche 7 bis 12.

## Claims

1. A method of identifying a filter, wherein the filter has at least one retentate side and at least one permeate side which are separated from one another by at least one filter medium, wherein the method comprises generating a pressure in a fluid, in particular in a liquid, on the retentate side or on the permeate side by means of at least one pressure source, in particular by means of at least one pump; switching off the pressure source; and the measurement of the pressure development in the fluid over time subsequent to the switching off of the pressure source.

2. A method in accordance with claim 1, **characterized in that** the filter is a dialyzer; and/or **in that** the filter medium is a semi-permeable membrane.

3. A method in accordance with one of the preceding claims, **characterized in that** the pressure development is only measured by means of a single pressure sensor, with provision preferably being made that the pressure is measured on that side (permeate side or retentate side) of the filter on which the pressure is generated by the pressure source.

4. A method in accordance with one of the preceding claims, **characterized in that** the pressure source increases the pressure in the fluid for so long until a specific pressure has been reached; and **in that** the pressure source is subsequently switched off.

5. A method in accordance with one of the preceding claims, **characterized in that** the retentate side and/or the permeate side of the filter is/are connected to one or more fluid lines, with one or more blocking valves being provided in these fluid lines; and **in that** these blocking valves are closed during the measurement of the pressure development over time in the fluid.

6. A method in accordance with one of the preceding claims, **characterized in that** the filter is completely filled with fluid before the application of the pressure.

7. A system of identifying a filter, wherein the filter has at least one retentate side and at least one permeate side that are separated from one another by at least one filter medium, wherein at least one pressure source, in particular at least one pump, is provided which is configured and arranged such that a pressure can be generated in a fluid, in particular in a liquid, on the retentate side or on the permeate side of the filter and comprising at least one pressure sensor which is arranged such that the pressure development over time in the fluid can be determined by means of the pressure sensor, with the system furthermore having at least one control unit which is connected to the pressure source and to the pressure sensor,
**characterized in that** the control unit is configured:
to control the pressure source such that the pressure in the fluid is increased until a specific pressure has been reached and subsequently to switch off the pressure source,
to carry out a measurement of the pressure development over time after the switching off of the pressure source, and
to draw a conclusion on a filter type or a filter characteristic based on this measurement.

8. A system in accordance with claim 7, **characterized in that** the filter is a dialyzer; and/or **in that** the filter medium is a semi-permeable membrane.

9. A system in accordance with claim 7 or claim 8, **characterized in that** only a single pressure sensor is present, with provision preferably being made that this pressure sensor is arranged on that side (permeate side or retentate side) of the filter on which the pressure can be generated by the pressure source.

10. A system in accordance with one of the claims 7 to 9, **characterized in that** no sensor is present for detecting mechanically or optically detectable filter features.

11. A system in accordance with one of the claims 7 to 10, **characterized in that** the retentate side and/or the permeate side of the filter is/are connected to one or more fluid lines, with one or more blocking valves being provided in these fluid lines, and with the control unit being configured such that it closes these blocking valves during the measurement of the pressure development over time in the fluid.

12. A system in accordance with claim 11, **characterized in that** the retentate side and the permeate side are each connected to an inflow line and to an outflow line; **in that** the pressure source is arranged in one of these lines; and **in that** the named blocking valves are located in the further lines.

13. A blood treatment device, in particular a dialysis machine, having at least one system in accordance with one of the claims 7 to 12.

## Revendications

1. Procédé d'identification d'un filtre, dans lequel le filtre comporte au moins un côté rétentat et au moins un côté perméat, qui sont séparés l'un de l'autre par au moins un milieu filtrant, le procédé comprenant la génération d'une pression dans un fluide, en particulier dans un liquide, du côté rétentat ou du côté perméat, au moyen d'au moins une source de pression, en particulier au moyen d'au moins une pompe, l'arrêt de la source de pression ainsi que la mesure, subséquente à l'arrêt de la source de pression, de l'évolution dans le temps de la pression dans le fluide.

2. Procédé selon la revendication 1, **caractérisé en ce que** le filtre est un dialyseur et/ou **en ce que** le milieu filtrant est une membrane semi-perméable.

3. Procédé selon l'une des revendications précédentes, **caractérisé en ce que** l'évolution de la pression est mesurée uniquement au moyen d'un seul capteur de pression, la pression étant de préférence prévue pour être mesurée du côté (côté perméat ou côté rétentat) du filtre duquel la pression a été générée par la source de pression.

4. Procédé selon l'une des revendications précédentes, **caractérisé en ce que** la source de pression augmente la pression dans le fluide jusqu'à ce qu'une pression déterminée soit atteinte et **en ce que** la source de pression est ensuite arrêtée.

5. Procédé selon l'une des revendications précédentes, **caractérisé en ce que** le côté rétentat et/ou le côté perméat du filtre est raccordé à un ou plusieurs conduits de fluide, une ou plusieurs soupapes d'arrêt étant disposées dans ces conduits de fluide et **en ce que** ces soupapes d'arrêt sont fermées pendant la mesure de l'évolution dans le temps de la pression dans le fluide.

6. Procédé selon l'une des revendications précédentes, **caractérisé en ce que** le filtre est complètement rempli de fluide avant l'application de la pression.

7. Système d'identification d'un filtre, le filtre comportant au moins un côté rétentat et au moins un côté perméat, qui sont séparés l'un de l'autre par au moins un milieu filtrant, au moins une source de pression, en particulier au moins une pompe, étant prévue, qui est conçue et agencée de telle manière qu'une pression peut être générée dans un fluide, en particulier dans un liquide, du côté rétentat ou du côté perméat du filtre, et comprenant au moins un capteur de pression, qui est disposé de telle manière que l'évolution dans le temps de la pression dans le fluide peut être déterminée au moyen du capteur de pression, le système comportant en outre au moins une unité de commande, qui est en liaison avec la source de pression ainsi qu'avec le capteur de pression,
**caractérisé en ce que** l'unité de commande est conçue pour :
commander la source de pression de telle manière que la pression dans le fluide est augmentée jusqu'à ce qu'une pression déterminée soit atteinte et arrêter ensuite la source de pression,
effectuer une mesure d'une évolution dans le temps de la pression après l'arrêt de la source de pression, et
déduire un type de filtre ou une caractéristique de filtre sur la base de cette mesure.

8. Système selon la revendication 7, **caractérisé en ce que** le filtre est un dialyseur et/ou **en ce que** le milieu filtrant est une membrane semi-perméable.

9. Système selon la revendication 7 ou 8, **caractérisé en ce qu'**il y a un seul capteur de pression, ce capteur de pression étant de préférence prévu pour être disposé du côté (côté perméat ou côté rétentat) du filtre duquel la pression peut être générée par la source de pression.

10. Système selon l'une des revendications 7 à 9, **caractérisé en ce qu'**il n'y a aucun capteur pour détecter des caractéristiques du filtre pouvant être détectées de manière mécanique ou optique.

11. Système selon l'une des revendications 7 à 10, **caractérisé en ce que** le côté rétentat et/ou le côté perméat du filtre est raccordé à un ou plusieurs conduits de fluide, une ou plusieurs soupapes d'arrêt étant disposées dans ces conduits de fluide et l'unité de commande étant conçue de telle manière qu'elle ferme ces soupapes d'arrêt pendant la mesure de l'évolution dans le temps de la pression dans le fluide.

12. Système selon la revendication 11, **caractérisé en ce que** le côté rétentat ainsi que le côté perméat sont raccordés respectivement à un conduit d'alimentation et à un conduit d'évacuation, **en ce que** la source de pression est disposée dans l'un de ces conduits et **en ce que** lesdites soupapes d'arrêt se trouvent dans les autres conduits.

13. Appareil de traitement du sang, en particulier appareil de dialyse comprenant au moins un système selon l'une des revendications 7 à 12.
